# EUROPEAN PATENT APPLICATION

(11) **EP 2 832 370 A1**
(43) Date of publication of application: **04.02.2015**
(21) Application number: 13768521.0
(22) Date of filing: 28.02.2013
(51) Int. Cl.: A61K 47/04, A61K 31/19, A61K 31/194, A61K 31/616, A61K 31/00, A61K 9/08, A61J 3/00

(54) **METHOD FOR PRODUCING DRUGS AND BIOLOGICALLY ACTIVE AGENTS**

(30) Priority: 29.03.2012 RU 2012112108
(71) Applicant: Postnov, Sergei Evgenievich, Moskovskaya obl., G. Zhukovsky 140180 (RU)
(72) Inventor: Postnov, Sergei Evgenievich, Moskovskaya obl., G. Zhukovsky 140180 (RU)
(74) Representative: Glawe, Delfs, Moll
(86) International application number: PCT/RU2013/000159
(87) International publication number: WO 2013/147645

(57) **Abstract**

The present invention relates to pharmaceutical industry and can find application in the production of medicinal and health-improvement agents, of compositions for medical purposes, of biologically active additives, of veterinary compositions, etc.

The technical result achieved lies in the obtaining of drugs and biologically active agents in which pharmaceutical ingredients are present in a reduced condition, stable in time, that involves their increase in affinity to the chemical form of the active ingredient (reduced condition), when it gets into biological fluids of a living organism (into blood, lymph, cellular and intracellular fluids).

Said technical result is obtained thanks to the fact that in the process for producing liquid-base drugs and biologically active agents, comprising dissolving in a liquid medium of at least one active pharmaceutical ingredient taken in a pharmaceutically active amount, as the liquid medium, use is made of an aqueous solution of potassium and sodium bicarbonates taken in a ratio corresponding to their mean content in the organism internal fluids. Preferably, after dissolving the sodium and potassium bicarbonates, the sodium ions to the potassium ions ratio in the liquid medium should be of (2-6): 1.

Said technical result is obtained as well by the fact that when producing compositions comprising more than one active pharmaceutical ingredient, an active pharmaceutical ingredient with an alkaline base is dissolved first, then an active pharmaceutical ingredient with an acid or neutral base or an agent demonstrating high biological activity is dissolved, the redox potential of liquid-base drugs and biologically active agents being preferably not more than 0 mV. In this case, the pharmaceutical ingredients of the composition can be introduced into the aqueous solution with the redox potential lowered to a value of no more than 0 mV, or initially all the pharmaceutical ingredients according to the formulation can be introduced into an aqueous solution and then the redox potential of the aqueous solution is lowered to a value of no more than 0 mV.

## Description

### Field of the invention

The present invention relates to pharmaceutical industry and can find application in the production of medicinal and health-improvement agents, of compositions for medicine, of biologically active additives, of veterinary compositions, etc.

### Background of the invention

One of the most important factors to control the parameters of reduction-oxidation reactions is the electronic activity measured as a redox potential (ROP). The ROP of the internal medium of humans has negative values within -30 to -130 millivolts (mV). For example, human plasma presents the ROP values of approx. -30 mV in arteries, and of approx. -120 mV in veins; intracellular fluid has the ROP value within -100 to-120 mV. It means that the internal fluid of a living organism has electron-donor properties, which fundamentally differentiates it from, for example, drinking water the ROP of which is usually within +100 to +400 mV. In particular, the artesian water ROP value is from approx. +200 to +350 mV, that of distilled water being equal to approx. +290 mV.

Said ROP differences of the human organism internal medium and of drinking water indicate that the electron activity in the reduction-oxidation reactions occurring in the human organism internal medium is shifted to reduction reactions while that of drinking water is shifted to oxidation reactions. If drinking water getting into the human organism has a ROP close to the ROP value of the human organism, the last does not practically spend energy to modify physical properties of water to adjust the electron activity. And this water is immediately assimilated for being biophysically compatible as to this parameter.

This phenomenon is particularly critical when water getting into the human organism is the base of drugs and biologically active agents and contains pharmaceutically active components the effect of which in some cases can differ from the predicted one. But these negative processes can be prevented if the water getting into the human organism, being the base of drugs and biologically active agents has the physical properties of the organism internal medium, i.e. presents reduction properties.

Depending on the ROP values, several main conditions met in aqueous solutions are distinguished:
- oxidation condition characterized by the ROP value of >+100 mV,
- transient reduction-oxidation condition determined by the ROP values of 0 to +100 mV.
- reduction condition characterized by the ROP value of <0 mV.

As it follows from the above stated, in the water acquired by the organism from outside, oxidation reactions prevail, and in the plant sap the transient condition is observed. At the same time, in a human organism, only a reduction reaction takes place. It means that in drugs made, for example, on the basis of distilled water, pharmaceutical ingredients are in an oxidized condition, and, as a result, after getting into a human organism, they will undergo inevitable chemical transformations such as the passage to a reduced state. In contrast to pharmaceutical production, these transformations will not occur in an isolated, specially prepared aqueous medium, but in blood plasma, in cellular and intracellular fluid, in lymph, i.e. in a medium where a large amount of organic and inorganic substances with high chemical activity exist. As a result, a part of the composition active substances will inevitably undergo chemical reactions, even without reaching their target, for example, a sore organ. And what is more, the process of substance transformation into a reduced form is not always possible if the organism is the object, for example, of acidosis. In this case, the percentage of reduction reactions drops, which is, along with the reaction of a part of the composition with the chemically active substances contained in the living organism fluids, in most of cases, a reason of inefficiency of the compositions.

Therefore, the main task is to make the active substances of drugs and biologically active agents, biologically active food additives, compositions for medical purposes, veterinary compositions, etc. enter the organism in a preliminarily reduced condition.

But at the same time, it is necessary to consider the fact that the main drawback of water having a negative ROP is the ROP instability in time. As a rule, water loses the negative ROP within only a few hours. At present, many countries widely use methods of stabilizing the negative ROP, based on addition of some types of amino acids in low concentrations, allowed to be used in infusion solutions, into the negative ROP water, see the patent RU2234945 C2. Furthermore, it is known to use sodium chloride as a stabilizer of aqueous solutions and of water-containing raw materials (V.I. Lobyshev, I.Iu. Petrushenko (Popova), V.I. Kiselev. Electrochemical activation of water. In the paper collection: Third International Symposium. Moscow, 2001, page 76).

Prior art discloses various processes for producing drugs, the closest of which is a process to obtain an artificial physiologic salt solution described in the patent RU Nº2352342,published on 20.04.2009, which uses, as liquid medium, some aqueous solution requiring addition of sodium chloride and/or potassium chloride with the potassium ions / sodium ions ratio from 1:4 to 1:8 in order to provide electrolytic reduction of the same.

A drawback of the above process resides in the fact that the treatment of water based on chemical activation in the presence of potassium and sodium chloride results in the separation, during said water activation procedure, of chlorine ions characterized by a high reactivity responsible for the chlorine ions reactions with substances dissolved in water, with the possibility of forming highly toxic compounds and free chlorine. In consequence, the technology of water treatment is complicated due to the need to eliminate both the free chlorine and the chlorine-containing compounds, together with the impossibility to obtain a drug having assigned and expected properties.

It is known a patent EP 1312261 B1 (TROY TECHNOLOGY CORPORATION, INC.) of 30.08.2006, describing an aqueous composition and a process of obtaining the same, the composition consisting of an active agent dissolved or suspended in water comprising a suitable buffer. Among other things, the buffer can comprise potassium salts, including potassium bicarbonate, and corresponding sodium salts. The use of the buffer enables to stabilize the end-use composition and to maintain pH of the medium, preventing by the same any active agent decomposition. However, the EP 1312261 B1 does not mention any ROP lowering but only describes the modification of pH of the medium. According to the claimed application, any solution stabilization over the hydrogen ion exponent pH, i.e. the characteristic of the hydrogen ions presence in a solution, is impossible, since the hydrogen ions can not be electron donors in reduction reactions for having no electrons, and it is not possible to characterize a solution having electron-donor properties with the hydrogen ion exponent pH.

### Summary of the invention

The object of the present invention is to provide an economical process for producing liquid drugs and biologically active agents to be introduced into a living organism under a reduced condition.

While solving the set task, a technical result is achieved that lies in the obtaining of drugs and biologically active agents in which pharmaceutical components are present in a reduced form, stable in time, that involves their increase in affinity to the chemical form of the active ingredient (reduced condition), when it gets into biological fluids of a living organism (into blood, lymph, cellular and intracellular fluids).

In the context of the object of the invention, as active pharmaceutical ingredient, one can understand any active agent of organic and/or inorganic nature used to obtain necessary results in diagnosis, treatment or prevention of various diseases.

Said technical result is obtained thanks to the fact that the process for producing liquid-base drugs and biologically active agents, comprising dissolving in a liquid medium of at least one active pharmaceutical ingredient taken in a pharmaceutically active amount, uses, as said liquid medium, an aqueous solution of potassium and sodium bicarbonates taken in a ratio corresponding to their mean content in the organism internal fluids. Preferably, after dissolving the sodium and potassium bicarbonates, the sodium ions to the potassium ions ratio in the liquid medium should be of (2-6): 1. This potassium and sodium ions ratio in an organism is well-known, it is generally accepted and does not find any objection in the Pharmaceutical Committee or in medical establishments. The use of potassium and sodium bicarbonates taken in the ratio of (2-6): 1, taken as ROP stabilizers in time, is explained by the fact that sodium and potassium cations play the most important part in the water-electrolyte balance regulation. In humans, the intracellular sodium content is 5 to 10 times lower that that of potassium. In blood, the ratio is opposite. The potassium content in blood plasma is of 4-5 mmol/l, whereas it is of 110-150 mmol/l in the cells (Pathological physiology of typical metabolic disturbances. Pathological physiology, edited by A.D. Ado and L.M. Ishimova, Moscow, Meditsina, 1980).

Said technical result is obtained as well by the fact that when producing compositions comprising more than one active pharmaceutical ingredient, an active pharmaceutical ingredient with an alkaline base is dissolved first, then an active pharmaceutical ingredient with an acid or neutral base or an agent demonstrating high biological activity is dissolved, the redox potential of liquid-base drugs and biologically active agents being preferably not more than 0 mV. In this case, the pharmaceutical ingredients of the composition can be introduced into the aqueous solution with the redox potential lowered to a value of no more than 0 mV, or initially all the pharmaceutical ingredients according to the formulation can be introduced into an aqueous solution and then the redox potential of the aqueous solution is lowered to a value of no more than 0 mV.

Studies demonstrated that if alkalis are introduced into water and then the ROP of the solution is lowered to get them reduced and to provide reducing power to the solution, any addition of acid ingredients to the last will not induce a neutralization reaction of the alkalis and acids introduced into the same, and vice-versa. This result enables to expand the technology of producing drugs and health-improving agents. The results of the studies show that simultaneous presence of alkalis and acids in their reduced condition leads to synergism. It means that the integral effect of interaction between two or more substances of different chemical nature being in a reduced condition and introduced into a solution is far from bringing to neutralize their properties but considerably surpasses the effect of every individual component or their bare sum, being introduced into a living organism separately or simultaneously in an oxidized condition.

A similar effect is observed when bicarbonates are introduced into water before lowering the ROP and then the alkalis and acids are added, following below described procedures. The presence of bicarbonates, according to our studies, intensifies the biological effect of ingredients. At the same time, lowering the ROP as an intermediate operation does not enable to provide a neutralization reaction in a wide pH range of 5.5 to 9.5. For example, it is possible to prepare a solution at pH=6 and ROP=9.0, when the acid medium will have electron-donor properties. Lowering the ROP at an intermediate stage of the composition preparation procedure enables to confer new biological properties to the produced compositions while keeping unchanged and even reinforcing their specific properties.

Besides, while producing compositions comprising more than one active pharmaceutical ingredient, initially at least one ingredient of the composition can be introduced into an aqueous solution, then the reduction-oxidation potential is reduced to a value of not more than 0 mV, and after that the other ingredients are added.

The process for producing compositions in the reduced condition of active substances can be carried out according to one of the following procedures:
1. Process for producing a drug composed of one active ingredient:
   a) sodium bicarbonate and potassium bicarbonate are dissolved in water in order to get the sodium ions to potassium ions ratio of 3:1.
   b) the reduction-oxidation potential of the solution obtained is lowered to a value from 0 mV to - 900 mV,
   c) the active ingredient of a drug or of a biologically active agent is dissolved in the solution obtained.
2. Process for producing a drug composed of several active ingredients:
   a) sodium bicarbonate and potassium bicarbonate are dissolved in water in order to get the sodium ions to potassium ions ratio of 3:1.
   b) the reduction-oxidation potential of the solution obtained is lowered to a value from 0 mV to - 900 mV,
   c) an acid (alkali) active ingredient of a drug or of a biologically active agent is dissolved in the solution obtained,
   d) the reduction-oxidation potential of the solution obtained is lowered to a value from 0 mV to - 900 mV,
   e) the acid (alkali) active ingredient of a drug or of a biologically active agent is dissolved in the solution obtained.

According to the claimed process, lowering water ROP can be carried out by any procedure known at present and providing the ROP value of < 0 mV, for example, with the method of electrochemical activation (V.M. Bakhir. Electrochemical activation, part 2, VNIIIMT. Moscow. 1992. P. 121-134), with the method of introducing molecular hydrogen (N.A.Aristova, I.M. Piskarev. Activation of molecular hydrogen dissolved in water. // "Water: chemistry and ecology", No 1 (2009), p. 27-32), with contactless methods (patent RU 2171232, Int. Cl. C02F 1/48, or Internationaler Kongress EURUMEDICA, Hannover, 3-7 of June 2010. Postnov S. «Boundary layer water and medicines on its basis - a new trend in biomedical technology»), and with other methods.

It is possible to lower the ROP by the method of "Electrochemical water activation" in the case when the ROP lowering is the first technological operation, i.e. before introducing active ingredients, since the electrochemical reactions occurring in water, while applying the present process, lead to the degradation of substances and/or create conditions for their active interaction; and furthermore, if the produced drugs are not foreseen to be introduced into the eyes, nose or in children.

### Embodiments of the invention

The possibility to pursue the invention is illustrated by the following examples:

### Example 1. Process for producing an oral cavity freshener or a medical composition for irrigating the oral cavity and nose mucous membrane.

The ROP is lowered by the method of water release from a boundary layer formed on a surface system at a low flow rate: Internationaler Kongress EURUMEDICA, Hannover, 3-7 of June, 2010. Postnov S. «Boundary layer water and medicines on its basis - a new trend in biomedical technology». In this example, the ROP lowering can be performed as well by other above mentioned processes, except the method of "Electrochemical water activation", since water with lowered ROP obtained with this method induces burns of the mucous membrane.

To water having the lowered ROP < 0 mV (for 10 ml of water), 0.2 mg/ml of potassium bicarbonate and 0.6 mg/ml of sodium bicarbonate are added. This aqueous solution is employed to moisten the nose and oral cavity mucous membrane, to recover the necessary moisture content level for a dry mucous membrane in the nasal cavity, to prevent formation of scabs, to favor mucus elimination in the nasal cavity when it is stuffed up, helping to recover breathing. It refreshes the oral cavity and attenuates the pains while swallowing on the background of an acute respiratory virus infection.

The data obtained testify that in 75-80% of cases, patients with urogenital diseases, sick of grippe and of an acute respiratory virus infection, as well as patients with tumors are sensible to the medical composition made in accordance with this procedure. Patients with genital sepsis and with papillomatosis have demonstrated sensitivity to this composition in 55-60% of cases (see Table 1).

At the same time, it has been experimentally shown that when bicarbonates are introduced into water without lowering the ROP, no therapeutic effect has been found out.

**Table 1**

| Therapeutic potential | | | |
|---|---|---|---|
| Disease | Number of patients (%) | | |
| | Drug composition | Cycloferonum | Ridostin |
| Virus of herpes catharrhalis-2 | 60 | 45 | 70 |
| Urogenital diseases | 80 | 35 | 50 |
| Grippe and acute respiratory virus infection | 80 | 60 | 60 |
| Papillomatosis | 55 | 50 | 60 |
| Tumors | 75 | 55 | 40 |

The above given example shows the fundamental difference of the bicarbonate addition of the present invention process from the process of dissolving the same in ordinary water.

### Example 2. Drug composition to moisten the nose and oral cavity mucous membrane in children younger than 3 years.

Composition: to 10 ml of water, it is added:
Sodium bicarbonate and/or potassium bicarbonate 0.001-1 g,
Succinic acid and/or ascorbic acid and/or citric acid 0.001-1 g.

For the above mentioned use purpose, it is necessary to carry out normalization of the hydrogen ion exponent pH, since the bicarbonates show a highly expressed alkali reaction. The production process is run as follows: To water having a classic ROP of approx. +300 mV, 0.2 mg/ml of potassium bicarbonate and 0.6 mg of sodium bicarbonate are added. After that, the solution ROP is lowered to the ROP of <0 mV. The ROP is lowered by releasing water from the boundary layer formed on the surface system at a low flow rate: Internationaler Kongress EURUMEDICA, Hannover, 3-7 of June, 2010. Postnov S. «Boundary layer water and medicines on its basis - a new trend in biomedical technology». In this example, the ROP can be lowered as well by other above mentioned processes, except the "Electrochemical water activation" method.

After ROP lowering, pH is normalized by succinic and/or ascorbic and/or citric acid to pH values of 7.4-8.4. If the potassium and sodium bicarbonates are added simultaneously with the addition of acid, a neutralization reaction will occur. Lowering the solution ROP after adding the bicarbonates enables to prevent any neutralization reaction in a successive technological operation.

In the present example, lowering the solution ROP at one of the intermediate stages of the technology is essential, since it enables to prevent any chemical reaction between the chemical substances of the composition, which in turn enables to keep the properties of the same.

### Example 3. Source of microelements: Beverage, biologically active additive, compositions for medical purposes or pharmaceutical compositions.

Composition: to 10 ml of water, it is added:
Sodium bicarbonate and/or potassium bicarbonate 0.001-1 g and/or:

| | |
|---|---|
| ascorbic acid | 0.001-1 g |
| sodium ascorbate | 0.001-0.2 g |
| calcium ascorbate | 0.001-0.2 g |
| potassium ascorbate | 0.001-0.2 g |
| magnesium ascorbate | 0.001-0.2 g |
| manganese ascorbate | 0.001-0.2 g |
| copper ascorbate | 0.001-0.2 g |
| zinc ascorbate | 0.001-0.2 g |
| chromium ascorbate | 0.001-0.2 g |
| chitosan oligosaccharide ascorbate | 0.001-0.2 g. |

ROP lowering is carried out like in Example 2.

In the present example, lowering the solution ROP to values lower than 0 mV after adding the bicarbonates and before adding the ascorbates is essential, since it enables to prevent any chemical neutralization reaction between these chemical substances of the composition, which in turn enables to keep the formulation of the same and to keep its properties.

### Example 4. Beverage, biologically active additive

Composition: to 10 ml of water, it is added:

| | |
|---|---|
| Sodium bicarbonate and/or potassium bicarbonate | 0.001-1 g |
| honey | 0.001-1 g |
| and/or: | |
| succinic acid | 0.001-1 g |
| ascorbic acid | 0.001-1 g |
| citric acid | 0.001-1 g. |

The ROP lowering is carried out according to one of the above mentioned processes.

### Example 5. Source of water-soluble vitamins: Beverage, biologically active additive, compositions for medical purposes or pharmaceutical compositions.

Composition: to 10 ml of water, it is added:

| | |
|---|---|
| Sodium bicarbonate and/or potassium bicarbonate | 0.001-1 g |
| and/or succinic acid | 0.001-1 g |
| ascorbic acid | 0.001-1 g |
| citric acid | 0.001-1 g |
| and/or | |
| Vitamin B₁ | 0.001-0.1 g |
| Vitamin B₂ | 0.001-0.1 g |
| Vitamin PP | 0.001-0.1 g |
| Vitamin B₃ | 0.001-0.1 g |
| Vitamin B₆ | 0.001-0.1 g |
| Vitamin H | 0.001-0.1 g |
| Vitamin B_{C} | 0.001-0.1 g |
| Vitamin B₁₂ | 0.001-0.1 g |
| Vitamin P | 0.001-0.1 g |
| Vitamin N | 0.001-0.1 g. |

The ROP value is lowered like in Example 2. In the present example, lowering the solution ROP to values lower than 0 mV after adding the bicarbonates and before adding the water-soluble vitamins is essential, since it enables to prevent any chemical neutralization reaction between the bicarbonates and the succinic (ascorbic, citric) acid, which in turn enables to introduce the vitamins into a solution with normalized pH. It enables to keep the formulation of the composition and to keep the vitamin properties, for example, like in a freshly pressed juice.

### Example 6. Antipyretic drug composition

Composition: to 10 ml of water, it is added:

| | |
|---|---|
| Sodium bicarbonate and/or potassium bicarbonate | 0.001-1 g |
| Acetyl salicylic acid | 0.001-1 g. |

The ROP value is lowered like in Example 2. In the present example, lowering the solution ROP to values lower than 0 mV after adding the bicarbonates and before adding the acetyl salicylic acid is essential, since it enables to prevent any chemical neutralization reaction between the bicarbonates and the acetyl salicylic acid. Furthermore, the liquid form enables to prevent any contact of the stomach walls with highly-concentrated acetyl salicylic acid, which in turn enables to prevent side effects and to introduce a composition into an organism, in which the active pharmaceutical ingredient is in a reduced condition.

### Example 7. Drug having an effect on metabolism.

Composition: to 10 ml of water, it is added:

| | |
|---|---|
| Sodium bicarbonate and/or potassium bicarbonate | 0.001-1 g |
| L-carnitine and/or its acylic derivatives | 0.001-1 g |
| and/or succinic acid | 0.001-1 g |
| ascorbic acid | 0.001-1 g |
| citric acid | 0.001-1 g. |

In the present example, lowering the solution ROP to values lower than 0 mV after adding the bicarbonates and before adding, for example, succinic acid is essential, since it enables to prevent any chemical neutralization reaction between the bicarbonates and the acid. At the same time, the succinic acid is a pH regulator for the composition. In this case, the pH value is important since L-carnitine is a vitamin-like amino acid, sensitive to that parameter.

### Example 8.

To some beverage or to a composition containing such substances like sweeteners and/or flavor intensifiers, in order to decrease the reduction-oxidation potential, 3% to 10% of water with lowered ROP of < - 100 mV are added. It means that the composition ROP lowering is carried out at a final stage of its production. In this case, the technological operation of ROP lowering is essential as regards its biological activity. An experiment as follows has been carried out: test tubes with some beverage, containing a flavor intensifier have been separated into two groups. The first group of test tubes has been charged with Protozoa (paramecia) and water in the amount of 3% of said liquid volume in each test tube. The second group of test tubes has been charged with paramecia and with 3% of the same water the ROP of which has been lowered to approx. -200 mV. After that, the population of Protozoa has been monitored for 48 hours. As controls, test tubes with water used to make the beverage and with paramecia have been used, but without any sweetener and without ROP lowering. In the test tubes where water without lowered ROP has been added, the paramecia population has decreased 2.2 times compared to that of controls after 12 hours. In the test tubes where water with lowered ROP has been added, the population of paramecia has increased 1.3 times compared to that of controls. It means that the negative effect due to the presence of a chemically synthesized substance (flavor intensifier) has been eliminated. The present experiment shows that such a technological operation like the water ROP lowering in the composition at the last production stage of the same can be essentially important.

### Example 9. Drug, antioxidant.

Composition: to 10 ml of water, it is added:

| | |
|---|---|
| Sodium bicarbonate and/or potassium bicarbonate | 0.001-0.5 g |
| quercetin | 0.001-0.05 g |
| diquercetin | 0.001-0.05 g |
| and/or | |
| succinic acid | 0.001-1 g |
| ascorbic acid | 0.001-1 g |
| citric acid | 0.001-1 g. |

In the present example, water with natural content of bicarbonates is used. Some selected acid, quercetin and/or diquercetin are dissolved in the same. The ROP of the solution is lowered to values inferior to 0 mV after adding all the active pharmaceutical ingredients. After the ROP lowering, they are in a reduced condition, which enables, besides the high-grade improvement of their effect onto the living organism, to increase even more the storage life of a composition in liquid state. The method of the ROP lowering in this example is one of the above mentioned, except the "Electrochemical activation".

### Example 10. Drug, immunostimulant composition and antivirus composition.

Composition: to 10 ml of water, it is added:

| | |
|---|---|
| Sodium bicarbonate and/or potassium bicarbonate | 0.001-0.5 g |
| Sodium ribonucleate (Ridostin) | 0.001 - 0.5g. |

Into water having classical ROP of approx. +300 mV, 0.2 mg/ml of potassium bicarbonate and 0.6 mg of sodium bicarbonate are introduced. Then, the solution ROP is lowered to the value of < 0 mV. In this example, the ROP is lowered by the method of water release from the boundary layer formed on a surface system at a low flow rate: Internationaler Kongress EURUMEDICA, Hannover, 3-7 of June, 2010. Postnov S. «Boundary layer water and medicines on its basis - a new trend in biomedical technology». The method of the ROP lowering in this example can be carried out as well by other above mentioned procedures, except the "Electrochemical water activation". After that, Ridostin is added.

Sodium ribonucleate is a well-known immunostimulant composition.

The Table 2 shows comparative results of its use both in monotherapy and in a solution made according to the claimed technology, against human grippe virus. The investigations have been carried out in vitro in human cells M-7.

**Table 2. Effect of sodium ribonucleate (Ridostin) in Example 10 on the reproduction of the grippe virus A/Aichi1/68 (H3N2) in human cells M-7 in vitro.**

| Virus titer (IgT 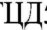 50) | Composition introduced along the virus | |
|---|---|---|
| | Ridostin **monotherapy** | Ridostin solution **Example 10** |
| 4.0 | 3.5 | 0.5 |
| | The Virus titer decreased 3 times | The Virus titer decreased 3162 times |

As written in the pharmacologic effect: "Sodium ribonucleate is active against some viruses". Example 10 has shown: The sodium ribonucleate solution produced in accordance with the claimed technology, in comparison with the monotherapy using the same pharmaceutical composition, **has become hundreds of times more efficient** against the human grippe virus.

It means that the sodium ribonucleate solution produced in accordance with the technology of the present invention has obtained a powerful antivirus effect against the human grippe virus.

### Example 11. Drug, immunostimulant, antivirus, antiinflammatory composition.

Composition: to 10 ml of water, it is added:

| | |
|---|---|
| Sodium bicarbonate and/or potassium bicarbonate | 0.001-0.5 g |
| Cycloferonum (methylglucamine acridone acetate) | 0.001 - 0.5g. |

Into water having a common ROP of approx. +300 mV, 0.2 mg/ml of potassium bicarbonate and 0.6 mg of sodium bicarbonate are introduced. Then, the solution ROP is lowered to the value of < 0 mV. In this example, the ROP is lowered by the method of water release from the boundary layer formed on a surface system at a low flow rate: Internationaler Kongress EURUMEDICA, Hannover, 3-7 of June, 2010. Postnov S. «Boundary layer water and medicines on its basis - a new trend in biomedical technology». The ROP lowering in this example can be carried out as well by other above mentioned processes, except the "Electrochemical water activation". Later on, Cycloferonum is added.

The comparative effect of Cycloferonum (monotherapy) and of a Cycloferonum solution produced in accordance with the present invention technology has been evaluated on monkeys and on volunteers currying the oncogenic virus SV40.

The blood count for the presence of the oncogenic virus SV40 has given the following results:
1. The efficiency of Cycloferonum (in monotherapy) against the oncogenic virus in humans and in monkeys does not exceed 20%. It means that no oncogenic virus has been detected in the blood of 20% of volunteers and of monkeys after a course of treatment.
2. The efficiency of the effect of a Cycloferonum solution produced in accordance with the methods of the present invention against the oncogenic virus SV40 in humans was as high as 50.6% (2.53-times increase), in monkeys it was 47.8% (2.4-times increase).

So, it has been shown that the production of pharmaceutical compositions in a reduced condition according to the technology of the present invention significantly increases the efficiency of their effect, enabling to improve considerably the quantitative indices.

### Example 12. Source of microelements: Beverage, biologically active additive, composition for medical purposes or pharmaceutical compositions.

Composition: to 10 ml of water, it is added:

| | |
|---|---|
| Sodium bicarbonate and/or potassium bicarbonate | 0.001-1g |
| and/or magnesium bicarbonate | 0.001-1g, |
| calcium bicarbonate | 0.001-1g, |
| and/or succinic acid | 0.001-1g |
| ascorbic acid | 0.001-1g |
| citric acid | 0.001-1g. |

In the present example, lowering the solution ROP to values lower than 0 mV after adding the bicarbonates and before adding an acid is essential, since it enables to prevent any chemical neutralization reaction between the bicarbonates and the succinic (ascorbic, citric) acid, which in turn enables to normalize the pH of the solution, when necessary. It enables to produce a composition both for adults and for children. This composition can be considered as a source of magnesium and/or of calcium.

## Claims

1. Process for producing a drug or a biologically active agent, comprising the stages as follows:
a) sodium bicarbonate and potassium bicarbonate are dissolved in water in order to get the sodium ions to potassium ions ratio in water of (2-6): 1,
b) the reduction-oxidation potential of the solution obtained is lowered to a value from 0 mV to - 900 mV,
c) the active ingredient of the drug or of the biologically active agent is dissolved in the solution obtained.

2. Process of claim 1, **characterized in that** the process comprises, for obtaining a composition including several active ingredients, the stages as follows:
a) sodium bicarbonate and potassium bicarbonate are dissolved in water in order to get the sodium ions to potassium ions ratio in water of (2-6): 1,
b) the reduction-oxidation potential of the solution obtained is lowered to a value from 0 mV to - 900 mV,
c) the active ingredient of the drug or of the biologically active agent is dissolved in the solution obtained,
d) the reduction-oxidation potential of the solution obtained is lowered to a value from 0 mV to - 900 mV,
e) the active alkali (acid) ingredient of the drug or of the biologically active agent is dissolved in the solution obtained.
